# EUROPÄISCHE PATENTSCHRIFT

(11) **EP 3 332 876 B1**
(45) Veröffentlichungstag und Bekanntmachung des Hinweises auf die Patenterteilung: **16.09.2020**
(21) Anmeldenummer: 16202741.1
(22) Anmeldetag: 07.12.2016
(51) Int. Cl.: B05B 15/00

(54) **VERFAHREN ZUR BESTIMMUNG DER PARTIKELGRÖSSEN UND/ODER DER MENGE AN PARTIKELN IN OVERSPRAY-AEROSOLEN BEI DER SPRITZAPPLIKATION VON BESCHICHTUNGSSTOFFEN**
METHOD FOR DETERMINING THE PARTICLE SIZES AND/OR THE AMOUNT OF PARTICLES IN OVERSPRAY AEROSOLS IN THE SPRAY APPLICATION OF COATING MATERIALS
PROCÉDÉ DE DÉTERMINATION DES TAILLES DE PARTICULES ET/OU DE LA QUANTITÉ DE PARTICULES DANS LA PERTE DE PEINTURE À LA PULVÉRISATION LORS DE L'APPLICATION PAR PULVÉRISATION DE PRODUITS DE REVÊTEMENT

(43) Veröffentlichungstag der Anmeldung: 13.06.2018
(73) Patentinhaber: DAW SE, 64372 Ober-Ramstadt (DE)
(72) Erfinder: Ottens, Stephan, 64404 Bickenbach (DE); Breivogel, Aaron, 64372 Ober-Ramstadt (DE)
(74) Vertreter: Metten, Karl-Heinz

(56) Entgegenhaltungen:
- EP-A1- 0 656 230
- EP-A2- 0 756 885
- WO-A1-97/12688
- DE-A1- 4 211 465
- DE-A1-102015 000 585
- US-A- 5 976 612

## Beschreibung

Die vorliegende Erfindung betrifft ein Verfahren zur Bestimmung der Partikelgrößen und/oder der Menge an Partikeln in Overspray-Aerosolen bei der Spritzapplikation von Beschichtungsstoffen. Des Weiteren betrifft die Erfindung die Verwendung des erfindungsgemäßen Verfahrens für die Entwicklung und Optimierung von Spritztechniken für die Applikation von Beschichtungsmassen, insbesondere von wasserbasierten oder lösemittelbasierten Beschichtungsmassen, und für die Entwicklung und Optimierung von für die Spritzapplikation vorgesehenen Beschichtungsmassen, insbesondere von wasserbasierten oder lösemittelbasierten Lacken.

Der Auftrag von Farben und Lacken mittels Rolle oder Pinsel ist seit langem im Malerhandwerk etabliert. Nicht selten sind die hierbei verwendeten Beschichtungsmaterialien auf die Beschichtungsmethode abgestimmt. Die Applikation von Beschichtungsstoffen mittels Pinsel oder Rolle ist jedoch zeit- und arbeitsintensiv, weswegen man in verstärktem Maße nach Alternativen sucht. Zu diesen Alternativen gehört die Spritzapplikation. Mit ihr können gegenüber dem Pinsel- oder Rollenauftrag deutlich höhere Arbeitsgeschwindigkeiten erreicht werden. Mitunter geht mit der Spritzapplikation eine Zeitersparnis im Bereich von 60 bis 80% einher.

Bei der Spritzapplikation wird regelmäßig ein Aerosol gebildet und für den Spritzauftrag genutzt. Man spricht daher auch von einem Spritzstrahl-Aerosol. In solchen Spritzstrahl-Aerosolen liegen feste und flüssige Teilchen häufig mit einer Teilchengröße im Bereich von bis zu etwa 150 µm vor. Nicht zuletzt aufgrund dieser Kleinteiligkeit und dem geringen Gewicht der Aerosolpartikel lassen sich diese Teilchen bei der Spritzapplikation auch außerhalb des eigentlichen Spritzstrahls, auch Haupt- oder Primärstrahl genannt, in der Raumluft feststellen, beispielsweise hinter der Spritzdüse. Man spricht bei dem außerhalb des Spritz-, Haupt- bzw. Primärstrahls vorliegenden Aerosol auch von dem sogenannten Overspray-Aerosol. Gegenüber dem Spritzstrahl- bzw. Primärstrahl-Aerosol liegt in dem Overspray-Aerosol ein höherer Anteil kleinerer Partikel vor. Wie das Aerosol des Spritzstrahls bzw. des Primärstrahls kann auch das Overspray-Aerosol neben flüssigen Partikeln auch feste Partikel enthalten. Als Bestandteil der Raumluft besteht die Gefahr, das Overspray-Aerosol einzuatmen. Auch kann das zeitlich verzögerte Absetzen der Overspray-Aerosolpartikel zu einer Verschmutzung von Oberflächen führen.

Um das Ausmaß der Bildung von Overspray-Aerosolen und damit auch die Güte des Spritzauftrags verlässlich und objektiv ermitteln zu können, steht dem Fachmann bislang nur ein begrenztes Arsenal an Nachweismethoden zur Fügung. Unter diesen stellt die Wägemethode die etablierteste Nachweismethode dar. Hierbei wird das Overspray-Aerosol durch ein tragbares Filter gesaugt. Die dabei aufgefangenen Aerosol-Partikel werden nach aufwendiger und zeitintensiver Konditionierung des Filters mittels Wägung bestimmt. Diese Methode gestattet es jedoch nicht, Aussagen über die Partikelgrößenverteilung von Overspray-Aerosolen zu machen. Letzteres wäre jedoch sehr wünschenswert. Denn bei der Untersuchung potentieller Gefahren für die Gesundheit sollte besonderes Augenmerk gerade auf die kleineren, leichten Aerosol-Partikel gelegt werden. Mit der Wägemethode lassen sich demgemäß die Güte weiterentwickelter Beschichtungsmassen für die Spritzapplikation und auch weiterentwickelte Spritzapplikationsmethoden nicht wirksam untersuchen.

Der vorliegenden Erfindung lag daher die Aufgabe zu Grunde, ein Verfahren zur Bestimmung von Overspray-Aerosolen zur Verfügung zu stellen, das nicht mit den geschilderten Nachteilen des Stands der Technik behaftet ist und das insbesondere die schnelle, zeitnahe und genaue Analyse von Overspray-Aerosolen gestattet. Der vorliegenden Erfindung lag zudem die Aufgabe zugrunde, ein Verfahren zu entwickeln, mit dem die Entwicklung und insbesondere die Geeignetheit von Beschichtungsmassen und Auftragsverfahren für die Spritzapplikation optimiert werden kann.

Demgemäß wurde ein Verfahren zur, insbesondere im Wesentlichen instantanen, Bestimmung der Partikelgrößen und/oder der Menge an Partikeln in, insbesondere der Partikelgrößenverteilung von, Overspray-Aerosolen bei der Spritzapplikation von, insbesondere flüssigen, Beschichtungsstoffen, umfassend die Schritte
- Zurverfügungstellung einer Beschichtungsmasse, einer Spritzvorrichtung enthaltend eine Spritzdüse, einer Ansaugvorrichtung, insbesondere eines Ansaugtrichters, mit einer Öffnung umfassend eine Ansaugfläche, einer Ansaugpumpe, eines Messsystems, enthaltend einen optischen Sensor, eines Probenschlauchs für die Zufuhr von Aerosolpartikeln von der Ansaugvorrichtung zu dem Messsystem, und einer Auswerteeinheit,
- Verbinden der Ansaugvorrichtung vermittels des Probenschlauches mit dem Messsystem,
- Verbinden des Messsystems mit der Auswerteeinheit,
- Positionieren der Spritzdüse der Spritzvorrichtung in einem, insbesondere abgeschlossenen, Messraum in einem ersten Abstand von einer mit der Beschichtungsmasse zu beschichtenden Substratoberfläche mit einem unteren Rand und einem oberen Rand,
- Positionieren der Ansaugvorrichtung in einem zweiten Abstand von der zu beschichtenden Substratoberfläche,
- Betätigen der Spritzvorrichtung, so dass die Beschichtungsmasse über die Spritzdüse in Form eines Aerosolstrahls, umfassend einen Aerosol-Hauptstrahl, auf die zu beschichtende Substratoberfläche appliziert wird,
- Ansaugen von Overspray-Aerosol über die Ansaugvorrichtung vermittels der Ansaugpumpe und Transport über den Probenschlauch zu dem Messsystem,
- Detektieren der Aerosolpartikel in dem Messsystem während einer Messdauer und weiterleiten der detektierten Signale an die Auswerteeinheit,
- Auswerten der detektierten Signale in der Auswerteeinheit, insbesondere hinsichtlich Größe und/oder Menge und besonders bevorzugt hinsichtlich der Partikelgrößenverteilung der Aerosol-Partikel im Overspray-Aerosol.

Die eingesetzten Beschichtungsstoffe sind grundsätzlich eingerichtet und ausgelegt, um für die Spritzapplikation verwendet werden zu können.

Das bei dem erfindungsgemäßen Verfahren zum Einsatz kommenden Messsystem umfasst vorzugsweise eine Lichtquelle, eine Durchflusszelle und einen Fotodetektor. Der Einsatz eines derartigen Messsystems erlaubt eine sehr kleinteilige und damit platzsparende Bauweise, so dass das erfindungsgemäße Verfahren für vielfältigste Anwendungen, sei es für sehr kleine oder sehr große Räume und/oder sehr kleine oder sehr große Substratoberflächen, geeignet ist.

Als Lichtquelle wird hierbei in einer zweckmäßigen Ausgestaltung auf eine Halbleiter-Laserdiode zurückgegriffen, vorzugsweise mit integrierter Optik zur Erzeugung paralleler Lichtstrahlen. Hierbei wird die Partikelgröße der Aerosolpartikel vorzugsweise mittels Streulichtmessung oder auf der Basis von Extinktionsmessungen bestimmt.

Besonders zuverlässige Resultate lassen sich mit dem erfindungsgemäßen Verfahren auch dadurch erzielen, dass die Ansaugvorrichtung in der Weise angeordnet wird, dass deren Öffnung außerhalb des Primärstrahls der Spritzdüse liegt. Auf diese Weise gelingt regelmäßig eine objektive, zuverlässige und vergleichbare Bestimmung der Partikelmenge und Partikelgrößenverteilung in den Overspray-Aerosolen.

Mit dem erfindungsgemäßen Verfahren lassen sich ohne weiteres Aerosolpartikel mit einer Partikelgröße im Bereich von 0,1 µm bis 40 µm, vorzugsweise im Bereich von 0,3 bis 20 µm, bestimmen.

Bei dem erfindungsgemäßen Verfahren wird in einer besonders zweckmäßigen Ausgestaltung darauf geachtet, dass die Spritzdüse der Spritzvorrichtung in der Weise ausgerichtet ist, dass der Hauptstrahl des Aerosolstrahls im Wesentlichen senkrecht auf die Substratoberfläche ausgerichtet ist. Der Hauptstrahl des Aerosolstrahls im Sinne der vorliegenden Erfindung umfasst regelmäßig denjenigen Bestandteil des Applikationsstrahls, insbesondere Partikelstrahlkegels, in dem die Partikeldichte am größten ist.

Für vielfältige Messapplikationen hat es sich als ausreichend erwiesen, die Spritzdüse in einem Abstand im Bereich von 0,1 bis 0,5 m, insbesondere im Bereich von 0,15 bis 0,3 m von der zu beschichtenden Substratoberfläche entfernt anzubringen. In einer alternativen Ausgestaltung kann auch vorgesehen werden, dass die Ansaugfläche der Ansaugvorrichtung in einem horizontalen Abstand im Bereich von 0,5 bis 3,0 m, insbesondere im Bereich von 1,0 bis 1,9 m, von der zu beschichtenden Substratoberfläche entfernt ist.

Auch hinsichtlich der Höhe der Anbringung des Absaugtrichters über dem Boden bzw. oberhalb des unteren Randes der zu beschichtenden Substratoberfläche steht dem Fachmann ein relativ großer Spielraum zur Verfügung. Es hat sich dabei als vorteilhaft erwiesen, wenn die Ansaugfläche des Ansaugtrichters sich auf einer Höhe im Bereich von 0 bis 2,0 m, vorzugsweise auf einer Höhe im Bereich von 0,5 bis 1,0 m, jeweils gerechnet vom unteren Rand der zu beschichtenden Substratoberfläche befindet, beispielsweise dem Boden eines Raumes. Diese Bedingungen reichen für viele Anwendungen völlig aus, um zu verlässlichen, reproduzierbaren Messergebnissen zu gelangen.

Des Weiteren hat es sich für viele Ausführungsvarianten des erfindungsgemäßen Verfahrens als vorteilhaft in Bezug auf eine verlässliche und genaue Bestimmung der Partikelgrößenverteilung des Overspray-Aerosols erwiesen, wenn der Messraum sich zu beiden Seiten des Aerosol-Hauptstrahls der Spritzdüse im Bereich von 1,0 bis 3,0 m, insbesondere im Bereich von 1,5 bis 2,5 m, erstreckt. Alternative kann dabei auch vorgesehen sein, dass der Messraum sich in Bezug auf die Spritzdüse jenseits der Ansaugfläche des Ansaugtrichters im Bereich von 1,0 bis 3,0 m, vorzugsweise im Bereich von 1,5 bis 2,5 m, erstreckt. Schließlich eignet sich das erfindungsgemäße Verfahren für die Vermessung der Partikelgrößenverteilung von Overspray-Aerosolen insbesondere auch in solchen Messräumen, die über eine Raumhöhe im Bereich von 2,0 bis 3,5 m, vorzuweise im Bereich von 2,25 bis 3,0 m, verfügen.

Insbesondere um zu vergleichbaren Ergebnissen zu gelangen, hat es sich als vorteilhaft erwiesen, die Temperatur im Messraum während der Messdauer konstant zu halten. Aus dem gleichen Grund versucht man vielfach auch, die relative Luftfeuchte im Messraum zu Beginn der Messdauer auf einen vorgegebenen Anfangswert einzustellen, um reproduzierbar zu vergleichbaren Messwerten zu gelangen.

Für die allermeisten Anwendungsfälle des erfindungsgemäßen Verfahrens hat es sich als ausreichend erwiesen, die Temperatur im Messraum im Bereich von 10 bis 22 °C, vorzugsweise im Bereich von 12 bis 20 °C, einzustellen. Zusätzlich oder alternativ kann dabei die relative Luftfeuchtigkeit im Messraum im Bereich von 0 bis 70%, vorzugsweise im Bereich von 40 bis 60%, gehalten werden.

Die Partikeldetektion im Sinne der vorliegenden Erfindung umfasst im Allgemeinen die Bestimmung der Anzahl und/oder der Größe der Partikel.

Des weiteren kann erfindungsgemäß vorgesehen sein, dass eine Bestimmung von Partikeln, insbesondere der Menge und/oder der Partikelgröße., gestartet wird, vorzugsweise über eine Zeitdauer im Bereich von 0,5 bis 3 Minuten, besonders bevorzugt im Bereich von 1,0 bis 2,0 Minuten, bevor die Spritzapplikation über die Spritzdüse auf die zu beschichtenden Substratoberfläche beginnt,

Mit dem erfindungsgemäßen Verfahren kann beispielsweise die Menge an Partikeln mit einer Partikelgröße im Bereich von 0,3 bis 20 µm ohne weiteres ermittelt werden. Auch gestattet das erfindungsgemäße Verfahren die Unterteilung des Partikelgrößenbereichs 0,3 bis 20 µm in mindestens zwei, insbesondere eine Vielzahl an Partikelgrößenintervallen, so dass die Menge an Partikeln pro Partikelgrößenintervall bestimmt werden kann. Optional können selbstverständlich auch die Menge an Partikeln mit einer Partikelgröße im Bereich größer 20 µm bestimmt werden.

Beispielsweise können die Partikelgrößenintervalle in einer Ausgestaltung wie folgt gewählt werden: im Bereich von 0,3 bis kleiner 0,5 µm, im Bereich von 0,5 bis kleiner 1,0 µm, im Bereich von 1,0 bis kleiner 2,0 µm, im Bereich von 2,0 bis kleiner 5,0 µm, im Bereich von 5,0 bis kleiner 10,0 µm, im Bereich von 10,0 bis kleiner 15,0 µm und im Bereich von 15,0 bis kleiner 20,0 µm sowie gegebenenfalls auch kleiner 0,3 µm und/oder größer 20,0 µm.

Für viele nach dem erfindungsgemäßen Verfahren durchgeführten Messungen hat es sich als vorteilhaft erwiesen, dass das während der Messung über die Ansaugfläche des Ansaugtrichters angesaugte Luftvolumen im Bereich von 0,1 bis 35,0 l/min, vorzugsweise im Bereich von 25,0 bis 30,0 l/min, liegt.

Besonders zuverlässige Aussagen über die Güte von Spritzbeschichtungsmassen und/oder Spritzverfahren erhält man insbesondere auch dadurch, dass die Bestimmung von Overspray-Aerosolpartikeln nach Beendigung der Spritzapplikation über die Spritzdüse für eine Dauer im Bereich von 0,5 bis 45 Minuten, vorzugsweise für eine Dauer im Bereich von 1,0 bis 30,0 Minuten, fortgesetzt wird.

Um während der Messung mit dem erfindungsgemäßen Verfahren möglichst praxisnahe Bedingungen zu simulieren, hat es sich vielfach als vorteilhaft erwiesen, dass der horizontale Abstand der Öffnung der Ansaugvorrichtung zur Spritzdüse mindestens 50 cm, insbesondere mindestens 75 cm, beträgt. Alternativ oder zusätzlich kann dabei vorgesehen sein, dass der Abstand der Öffnung der Ansaugvorrichtung zu der kürzesten Verbindungslinie zwischen Spritzdüse und der zu beschichtenden Substratoberfläche mindestens 50 cm, insbesondere mindestens 75 cm, beträgt.

Die erfindungsgemäßen Verfahren eignen sich in besonderer Weise für die Entwicklung und/oder Optimierung von Spritztechniken für die Applikation von Beschichtungsmassen. Beschichtungsmassen, die mit dem erfindungsgemäßen Verfahren hinsichtlich ihres Overspray-Anteils untersucht werden können, umfassen sowohl wasserbasierte wie auch lösemittelbasierte Beschichtungsmassen. Darüber hinaus eignet sich das erfindungsgemäße Verfahren für die Entwicklung und/oder Optimierung von für die Spritzapplikation vorgesehenen Beschichtungsmassen, insbesondere von wasserbasierten oder lösemittelbasierten Lacken.

Mithilfe des erfindungsgemäßen Verfahrens gelingt es in besonders effektiver Weise, Spritztechniken und/oder Beschichtungsmasse zu entwickeln bzw. zu optimieren, so dass bei Spritzapplikation die Menge an Overspray-Aerosolen reduziert ist.

Besonders vorteilhaft ist auch, dass mit dem erfindungsgemäßen Verfahren, das hinsichtlich Menge an Sprühpartikeln mit einer bestimmten Partikelgröße und auch Partikelgrößenverteilung das ausgewertete Ergebnis unverzüglich, d.h. instantan vorliegt. Auch kann auf den Einsatz von Filtern zur Mengenbestimmung von Sprühnebelpartikeln komplett verzichtet werden. Hiermit geht eine signifikante Zeit- und Arbeitsersparnis einher. Auch können mit dem erfindungsgemäßen Verfahren Tests zur Optimierung von Sprühapplikationsmethoden bzw. Spritzbeschichtungsmassen wesentlich kosteneffizienter durchgeführt werden. Auf diese Weise gelangt man mit dem erfindungsgemäßen Verfahren zu optimierten Spritzapplikationen und Spritzbeschichtungsmassen, die es aufgrund ihres geringen bzw. vemachlässigbaren Overspray-Anteils nicht mehr erforderlich machen, Flächen, die nicht mit dem Beschichtungsmaterial versehen werden sollen, abzukleben oder abzudecken. Der Beitrag, den das erfindungsgemäße Verfahren bei der Optimierung von Spritzbeschichtungsmassen und Spritzmethoden leistet, ist auch deswegen nicht gering zu achten, als die feinverteilten Sprühnebel des Overspray-Anteils regelmäßig sehr schnell austrocknen und sich als sehr leichtes Pulver großflächig verteilen und absetzen. Überdies kann man mit dem erfindungsgemäßen Verfahren auf verlässliche Weise zu solchen Spritzbeschichtungsmassen und Spritzmethoden gelangen, die im Overspray-Anteil über eine reduzierte Anzahl lungengängiger Partikel verfügen. Da bei neu entwickelten Beschichtungsmassen häufig nicht vorherzusehen ist, ob diese über einen geringen oder möglicherweise sogar sehr großen Overspray-Anteil verfügen, kann bislang eine ausgeprägte Exposition des die Beschichtungsmasse auftragenden Bedienpersonals nicht vermieden werden. Das erfindungsgemäße Verfahren ermöglicht es hingegen, sämtliche in dem Messraum befindlichen Komponenten, einschließlich der Spritzdüse, von außerhalb des Messraums zu bedienen. Damit kann die mögliche Gefährdung des Bedienpersonals vollständig ausgeschlossen werden.

Das erfindungsgemäße Verfahren liefert eine reproduzierbare Methode zur quantitativen Bestimmung von Overspray-Aerosolen, die bei der Spritzapplikation von Beschichtungsstoffen entstehen und die sich außerhalb des direkten Spritzstrahls befinden. Das erfindungsgemäße Verfahren eignet sich insbesondere für die objektive Beurteilung von Overspray-Aerosolen in der Raumluft bei der Spritzapplikation von Lacken. Ferner kann man mit dem erfindungsgemäßen Verfahren Overspray-Aerosole hinsichtlich Anzahl und Größe der darin vorliegenden Aerosolpartikel, insbesondere instantan, quantifizieren. Auch ermöglicht das erfindungsgemäße Verfahren den Vergleich von Overspray-Aerosolen von mindestens zwei, insbesondere einer Vielzahl an Beschichtungsmassen, welche für die Spritzapplikation geeignet sind, insbesondere von wasserbasierten oder lösemittelbasierten Lacken.

Weitere Merkmale und Vorteile der Erfindung ergeben sich aus der nachfolgenden Beschreibung, in der ein Ausführungsbeispiel der Erfindung anhand schematischer Zeichnungen beispielhaft erläutert wird, ohne jedoch dadurch die Erfindung zu beschränken. Dabei zeigt:

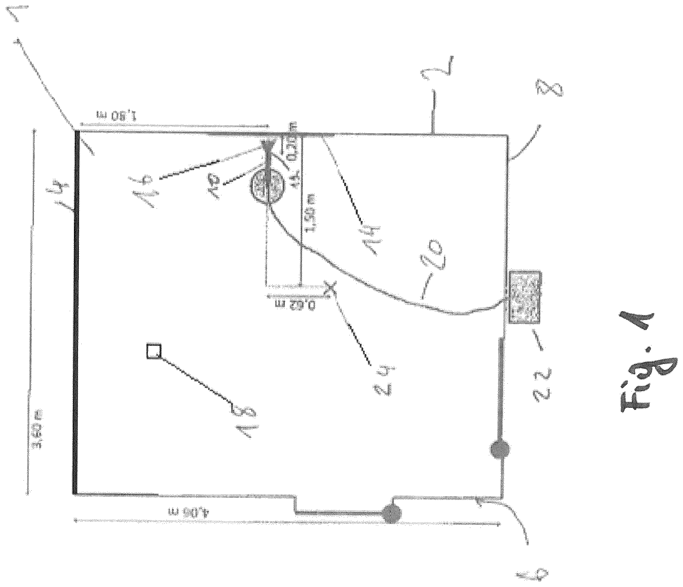

- Figur 1: eine schematische Darstellung der Messanordnung, mit der das erfindungsgemäße Verfahren durchgeführt werden kann.

In einem Messraum 1 mit vier Wänden 2, 4, 6 und 8, die im Wesentlichen eine rechteckige Grundform bilden, ist eine Spritzpistole 10 mit einer Spritzdüse 12 benachbart zu der Wand 2 mit einer beschichtbaren Fläche 14 angebracht. Die Spritzdüse 12 befindet sich in einem kürzesten Abstand von etwa 20 cm von der beschichtbaren Fläche 14 der Wand 2 entfernt. In dem dargestellten Messraum 1 erstreckt sich die beschichtbare Fläche 14 bis zum Boden. Die Spritzdüse 12 befindet sich, gerechnet vom Boden, auf einer Höhe von etwa 1,5 m. Die Spritzpistole samt Spritzdüse ist derart auf die beschichtbare Oberfläche 14 ausgerichtet, dass der Kernstrahl des kegelförmigen Primärstrahls 16 im Wesentlichen senkrecht auf die Oberfläche der Wand 2 auftrifft. Der dargestellte Messraum 1 verfügt über eine Raumhöhe von 2,45 m. Die Länge der sich gegenüberliegenden Wände 2 und 6 liegt bei etwa 4,1 m und die Länge der sich gegenüberliegenden Wände 4 und 8 bei etwa 3,6 m. Die Spritzpistole 10 ist in dem Messraum 1 in der geschilderten Anordnung fixiert. Auch ist die Spritzpistole derart ausgelegt und eingerichtet, dass sie von außerhalb des Messraums 1 bedient, d.h. ein- und ausgeschaltet werden kann. Hierfür wird die Schlauchverbindung 20 von der Spritzpistole bis zu einer außerhalb des Messraums 1 gelegenen Einspeisevorrichtung 22 geführt. In dem Messraum 1 ist beabstandet von der Spritzdüse 12 ein Temperatur- und Luftfeuchtemesser 18 angebracht. Beabstandet von der zu beschichtenden Fläche 14 wie auch von der Spritzdüse 12 ist hinter der Spritzpistole 10 eine Ansaugvorrichtung umfassend einen Ansaugtrichter 24 angebracht. Der Ansaugtrichter weist dabei einen Abstand von etwa 1,5 m von der zu beschichtenden Oberfläche 14 auf und ist in Bezug auf die Spritzdüse etwa 0,6 m seitlich zu dieser versetzt angeordnet. Es hat sich für viele Anwendungen als pragmatisch erwiesen, den Ansaugtrichter nicht direkt am Boden des Messraums 1 anzubringen, sondern beabstandet von diesem, beispielsweise in einer Höhe von 0,75 m. Die über den Ansaugtrichter eingesaugten Partikel des Oversprays der Spritzapplikation werden einem Messsystem enthaltend eine Lichtquelle, eine Durchflusszelle und einen Fotodetektor zugeführt. Hierbei kann sich das Messsystem innerhalb oder außerhalb des Messraums 1 befinden. Die detektierten Daten können dann z.B. einer außerhalb des Messraums vorliegenden Auswerteeinheit zugeführt werden (nicht abgebildet).

Mit der beschriebenen Messanordnung lassen sich Overspray-Aerosole, beispielsweise von Lacken, wie sie bei der Spritzapplikation entstehen, quantitativ analysieren. Selbstverständlich haben die Ausmaße des Messraums sowie die Messparameter nicht notwendigerweise den vorangehend dargestellten Werten zu entsprechen. Vielmehr kommt es für die Herbeiführung vergleichbarer Ergebnisse darauf an, dass der Messraum und die verwendeten Messparameter im Wesentlichen konstant gehalten werden, vorzugsweise immer dieselben sind. Denn auf diese Weise können unterschiedliche Beschichtungssysteme objektiv miteinander verglichen werden. Wie sich gezeigt hat, kann auf diese Weise sichergestellt werden, dass sich die Resultate unabhängig vom Anwender zuverlässig reproduzieren lassen. Für viele Anwendungen hat es sich als vorteilhaft erwiesen, wenn die Temperatur im Messraum während der Messung im Bereich von 12 bis 18 °C gehalten wird und wenn die relative Luftfeuchte zu Beginn der Messung im Bereich von etwa 50 bis 64 % liegt. Auch hat es sich für viele Anwendungen als vorteilhaft erwiesen, wenn vor Beginn der Spritzapplikation die Ansaugvorrichtung bereits eingeschaltet und die Detektion aufgenommen wird, beispielsweise etwa 1 bis 2 Minuten vor Beginn der Spritzapplikation. Das von außerhalb des Messraums bedienbare Spritzgerät kann sodann eingeschaltet und für beispielsweise einen Zeitraum von 3 bis 8 Minuten, z.B. 5 Minuten, in dem eingeschalteten Zustand belassen werden. Auch hat es sich für viele Messungen als sehr vorteilhaft erwiesen, wenn die Auswerte- und Detektionsvorrichtungen auch nach Beenden der Spritzapplikation für einen bestimmten Zeitraum eingeschaltet bleiben, beispielsweise über eine Zeitdauer von 10 bis 20 Minuten, zum Beispiel 14 Minuten.

Mit dem erfindungsgemäßen Verfahren gelingt eine instantane Quantifizierung von Overspray-Aerosolen hinsichtlich Anzahl und Größe der darin vorliegenden Aerosolpartikel. Auch gestattet es das erfindungsgemäße Messverfahren erstmals, Beschichtungsmassen anhand der Partikelgrößenverteilung der Overspray-Aerosole zu charakterisieren und miteinander zu vergleichen. Dies ist in der Maler- und Bautenbranche ein neuer Ansatz. Insbesondere ist es auch möglich, Beschichtungsmassen, beispielsweise Lacksysteme, dahingehend zu unterscheiden, ob sie zu viel oder wenig Overspray-Bildung neigen.

## Patentansprüche

1. Verfahren zur, insbesondere im Wesentlichen instantanen, Bestimmung der Partikelgröße und/oder der Menge an Aerosol-Partikeln, insbesondere der Partikelgrößenverteilung von Aerosol-Partikeln, in Overspray-Aerosolen bei der Spritzapplikation von Beschichtungsstoffen, umfassend die Schritte
- Zurverfügungstellung einer Beschichtungsmasse, einer Spritzvorrichtung enthaltend eine Spritzdüse, einer Ansaugvorrichtung, insbesondere eines Ansaugtrichters, mit einer Öffnung umfassend eine Ansaugfläche, einer Ansaugpumpe, eines Messsystems, enthaltend einen optischen Sensor, eines Probenschlauchs für die Zufuhr von Aerosolpartikeln von der Ansaugvorrichtung zu dem Messsystem, und einer Auswerteeinheit,
- Verbinden der Ansaugvorrichtung vermittels des Probenschlauches mit dem Messsystem,
- Verbinden des Messsystems mit der Auswerteeinheit,
- Positionieren der Spritzdüse der Spritzvorrichtung in einem, insbesondere abgeschlossenen, Messraum in einem ersten Abstand von einer mit der Beschichtungsmasse zu beschichtenden Substratoberfläche mit einem unteren Rand und einem oberen Rand,
- Positionieren der Ansaugvorrichtung in einem zweiten Abstand von der zu beschichtenden Substratoberfläche,
- Betätigen der Spritzvorrichtung, so dass die Beschichtungsmasse über die Spritzdüse in Form eines Aerosolstrahls, umfassend einen Aerosol-Primärstrahl, auf die zu beschichtende Substratoberfläche appliziert wird,
- Ansaugen von Overspray-Aerosol über die Ansaugvorrichtung vermittels der Ansaugpumpe und Transport von Aerosol-Partikeln des Overspray-Aerosols über den Probenschlauch zu dem Messsystem,
- Detektieren der Aerosolpartikel in dem Messsystem während einer Messdauer und Weiterleiten der detektierten Signale an die Auswerteeinheit,
- Auswerten der detektierten Signale in der Auswerteeinheit.

2. Verfahren nach Anspruch 1, **dadurch gekennzeichnet, dass**
das Messsystem eine Lichtquelle, eine Durchflusszelle und einen Fotodetektor umfasst.

3. Verfahren nach Anspruch 2, **dadurch gekennzeichnet, dass**
die Lichtquelle eine Halbleiter-Laserdiode umfasst oder darstellt, vorzugsweise mit integrierter Optik zur Erzeugung paralleler Lichtstrahlen.

4. Verfahren nach einem der vorangehenden Ansprüche, **dadurch gekennzeichnet, dass** die Ansaugvorrichtung in der Weise angeordnet wird, dass deren Öffnung außerhalb des Primärstrahls der Spritzdüse liegt.

5. Verfahren nach einem der vorangehenden Ansprüche, **dadurch gekennzeichnet, dass** Aerosolpartikel mit einer Partikelgröße im Bereich von 0,1 µm bis 40 µm, vorzugsweise im Bereich von 0,3 bis 20 µm, bestimmt werden.

6. Verfahren nach einem der vorangehenden Ansprüche, **dadurch gekennzeichnet, dass** die Bestimmung der Partikelgröße der Aerosolpartikel auf dem Prinzip der Streulichtmessung oder dem Prinzip der Extinktion basiert.

7. Verfahren nach einem der vorangehenden Ansprüche, **dadurch gekennzeichnet, dass** die Spritzdüse der Spritzvorrichtung in der Weise ausgerichtet ist, dass der Primärstrahl des Aerosolstrahls im Wesentlichen senkrecht auf die Substratoberfläche ausgerichtet ist.

8. Verfahren nach einem der vorangehenden Ansprüche, **dadurch gekennzeichnet, dass** die Spritzdüse in einem Abstand im Bereich von 0,1 bis 0,5 m, insbesondere im Bereich von 0,15 bis 0,3 m, von der zu beschichtenden Substratoberfläche entfernt ist und/oder dass
die Ansaugfläche der Ansaugvorrichtung in einem horizontalen Abstand im Bereich von 0,5 bis 3,0 m, insbesondere im Bereich von 1,0 bis 1,9 m, von der zu beschichtenden Substratoberfläche entfernt ist und/oder dass
die Ansaugfläche des Ansaugtrichters auf einer Höhe im Bereich von 0 bis 2,0 m, vorzugsweise auf einer Höhe im Bereich von 0,5 bis 1,0 m, jeweils gerechnet vom unteren Rand der zu beschichtenden Substratoberfläche liegt, und/oder dass der Messraum sich zu beiden Seiten des Aerosol-Primärstrahls der Spritzdüse im Bereich von 1,0 bis 3,0 m, insbesondere im Bereich von 1,5 bis 2,5 m, erstreckt und/oder dass
der Messraum in Bezug auf die Spritzdüse sich jenseits der Ansaugfläche des Ansaugtrichters im Bereich von 1,0 bis 3,0 m, vorzugsweise im Bereich von 1,5 bis 2,5 m, erstreckt und/oder dass
der Messraum eine Raumhöhe im Bereich von 2,0 bis 3,5 m, vorzuweise im Bereich von 2,25 bis 3,0 m, aufweist.

9. Verfahren nach einem der vorangehenden Ansprüche, **dadurch gekennzeichnet, dass** die Temperatur im Messraum während der Messdauer konstant gehalten wird.

10. Verfahren nach einem der vorangehenden Ansprüche, **dadurch gekennzeichnet, dass** die relative Luftfeuchte im Messraum zu Beginn der Messdauer auf einen vorgegebenen Anfangswert eingestellt wird.

11. Verfahren nach einem der vorangehenden Ansprüche, **dadurch gekennzeichnet, dass** die Temperatur im Messraum im Bereich von 10 bis 22 °C, vorzugsweise im Bereich von 12 bis 20 °C, liegt und/oder dass die relative Luftfeuchtigkeit im Messraum, insbesondere zu Beginn der Messdauer, im Bereich von 0 bis 70%, vorzugsweise im Bereich von 40 bis 60%, liegt.

12. Verfahren nach einem der vorangehenden Ansprüche, **dadurch gekennzeichnet, dass** Detektion von Partikeln die Bestimmung der Anzahl und/oder der Größe der Partikel umfasst.

13. Verfahren nach einem der vorangehenden Ansprüche, **dadurch gekennzeichnet, dass** die Detektion von Aerosol-Partikeln, insbesondere der Menge und/oder der Partikelgröße, gestartet wird, vorzugsweise über eine Zeitdauer im Bereich von 0,5 bis 3 Minuten, besonders bevorzugt im Bereich von 1,0 bis 2,0 Minuten, bevor die Spritzapplikation über die Spritzdüse auf die zu beschichtenden Substratoberfläche beginnt.

14. Verfahren nach einem der vorangehenden Ansprüche, **dadurch gekennzeichnet, dass** die Menge an Aerosol-Partikeln mit einer Partikelgröße im Bereich von 0,3 bis 20 µm ermittelt wird oder dass der Partikelgrößenbereich 0,3 bis 20 µm in mindestens zwei, insbesondere eine Vielzahl an Partikelgrößenintervallen unterteilt und die Menge an Partikeln pro Partikelgrößenintervall bestimmt wird und/oder dass die Menge an Partikeln mit einer Partikelgröße im Bereich größer 20 µm bestimmt wird.

15. Verfahren nach einem der vorangehenden Ansprüche, **dadurch gekennzeichnet, dass** das während der Messung über die Ansaugfläche des Ansaugtrichters angesaugte Luftvolumen im Bereich von 0,1 bis 35,0 l/min, vorzugsweise im Bereich von 25,0 bis 30,0 l/min, liegt.

16. Verfahren nach einem der vorangehenden Ansprüche, **dadurch gekennzeichnet, dass** die Detektion von Aerosol-Partikeln des Overspray-Aerosols nach Beendigung der Spritzapplikation über die Spritzdüse für eine Dauer im Bereich von 0,5 bis 45 Minuten, vorzugsweise für eine Dauer im Bereich von 1,0 bis 30,0 Minuten, fortgesetzt wird.

17. Verfahren nach einem der vorangehenden Ansprüche, **dadurch gekennzeichnet, dass** der horizontale Abstand der Öffnung der Ansaugvorrichtung zur Spritzdüse mindestens 50 cm, insbesondere mindestens 75 cm, und/oder dass der, insbesondere kürzeste, Abstand der Öffnung der Ansaugvorrichtung zu der kürzesten Verbindungslinie zwischen Spritzdüse und der zu beschichtenden Substratoberfläche mindestens 50 cm, insbesondere mindestens 75 cm, beträgt.

18. Verwendung des Verfahrens gemäß einem der vorangehenden Ansprüche für die Entwicklung und/oder Optimierung von Spritztechniken und/oder Spritzvorrichtungen für die Applikation von Beschichtungsmassen, insbesondere von wasserbasierten oder lösemittelbasierten Beschichtungsmassen, und/oder für die Entwicklung und/oder Optimierung von für die Spritzapplikation vorgesehenen Beschichtungsmassen, insbesondere von wasserbasierten oder lösemittelbasierten Lacken, und/oder für die, insbesondere instantane, Quantifizierung von Overspray-Aerosolen hinsichtlich Anzahl und Größe der darin vorliegenden Aerosolpartikel und/oder für den Vergleich der Overspray-Aerosole von mindestens zwei, insbesondere einer Vielzahl an Beschichtungsmassen, welche für die Spritzapplikation geeignet sind, insbesondere von wasserbasierten oder lösemittelbasierten Lacken.

19. Verwendung nach Anspruch 18, **dadurch gekennzeichnet, dass**
die Entwicklung und/oder Optimierung von Spritztechniken und/oder Spritzvorrichtungen und/oder von Beschichtungsmassen die Reduzierung der Menge an Overspray-Aerosolen umfasst.

## Claims

1. A method for, in particular substantially instantaneously, determining the particle size and/or the amount of aerosol particles, in particular the particle size distribution of aerosol particles, in overspray aerosols during the spray application of coating materials, comprising the steps of
- supplying a coating composition, a spray device containing a spray nozzle, a suction device, in particular a suction funnel, having an opening comprising a suction surface, a suction pump, a measuring system, containing an optical sensor, a specimen tube for feeding aerosol particles from the suction device to the measuring system, and an evaluation unit,
- connecting the suction device by means of the specimen tube to the measuring system,
- connecting the measuring system to the evaluation unit,
- positioning the spray nozzle of the spray device in an, in particular closed, measuring room at a first distance from a substrate surface to be coated with the coating composition having a lower edge and an upper edge,
- positioning the suction device at a second distance from the substrate surface to be coated,
- actuating the spray device such that the coating composition is applied via the spray nozzle in the form of an aerosol jet, comprising an aerosol primary jet, to the substrate surface to be coated,
- sucking in overspray aerosol via the suction device by means of the suction pump and transporting aerosol particles of the overspray aerosol via the specimen tube to the measuring system,
- detecting the aerosol particles in the measuring system during a measuring period and forwarding the detected signals to the evaluation unit,
- evaluating the detected signals in the evaluation unit.

2. The method according to Claim 1, **characterized in that**
the measuring system comprises a light source, a flow cell and a photo detector.

3. The method according to Claim 2, **characterized in that**
the light source comprises or is a semiconductor laser diode, preferably having integrated optics for producing parallel rays of light.

4. The method according to any one of the preceding claims, **characterized in that**
the suction device is arranged in such a way that its opening lies outside the primary jet of the spray nozzle.

5. The method according to any one of the preceding claims, **characterized in that** aerosol particles having a particle size in the range of 0.1 µm to 40 µm, preferably in the range of 0.3 to 20 µm, are determined.

6. The method according to any one of the preceding claims, **characterized in that**
the determination of the particle size of the aerosol particles is based on the principle of light-scattering measurement or the principle of extinction.

7. The method according to any one of the preceding claims, **characterized in that**
the spray nozzle of the spray device is aligned in such a way that the primary jet of the aerosol jet is substantially aligned perpendicularly to the substrate surface.

8. The method according to any one of the preceding claims, **characterized in that**
the spray nozzle is at a distance in the range of 0.1 to 0.5 m, in particular in the range of 0.15 to 0.3 m, from the substrate surface to be coated; and/or
the suction surface of the suction device is at a horizontal distance in the range of 0.5 to 3.0 m, in particular in the range of 1.0 to 1.9 m, from the substrate surface to be coated, and/or
the suction surface of the suction funnel lies at a height in the range of 0 to 2.0 m, preferably at a height in the range of 0.5 to 1.0 m, in each case calculated from the lower edge of the substrate surface to be coated, and/or
the measuring room extends on both sides of the aerosol primary jet of the spray nozzle in the range of 1.0 to 3.0 m, in particular in the range of 1.5 to 2.5 m, and/or with respect to the spray nozzle, the measuring room extends on the other side of the suction surface of the suction funnel in the range of 1.0 to 3.0 m, preferably in the range of 1.5 to 2.5 m, and/or
the measuring room has a ceiling height in the range of 2.0 to 3.5 m, preferably in the range of 2.25 to 3.0 m.

9. The method according to any one of the preceding claims, **characterized in that**
the temperature in the measuring room is kept constant during the measuring period.

10. The method according to any one of the preceding claims, **characterized in that** the relative air humidity in the measuring room at the start of the measuring period is adjusted to a predefined initial value.

11. The method according to any one of the preceding claims, **characterized in that**
the temperature in the measuring room lies in the range of 10 to 22°C, preferably in the range of 12 to 20°C, and/or
the relative air humidity in the measuring room, in particular at the start of the measuring period, lies in the range of 0 to 70%, preferably in the range of 40 to 60%.

12. The method according to any one of the preceding claims, **characterized in that**
the detection of particles comprises the determination of the number and/or the size of the particles.

13. The method according to any one of the preceding claims, **characterized in that**
the detection of aerosol particles, in particular of the amount and/or of the particle size, is started, preferably over a period of time in the range of 0.5 to 3 minutes, particularly preferably in the range of 1.0 to 2.0 minutes, before the spray application via the spray nozzle onto the substrate surface to be coated begins.

14. The method according to any one of the preceding claims, **characterized in that**
the amount of aerosol particles having a particle size in the range of 0.3 to 20 µm is established, or the particle size range 0.3 to 20 µm is divided into at least two, in particular a multiplicity of, particle size intervals, and the amount of particles per particle size interval is determined, and/or the amount of particles having a particle size in the range greater than 20 µm is determined.

15. The method according to any one of the preceding claims, **characterized in that**
the air volume sucked in during the measurement via the suction surface of the suction funnel lies in the range of 0.1 to 35.0 l/min, preferably in the range of 25.0 to 30.0 l/min.

16. The method according to any one of the preceding claims, **characterized in that**
the detection of aerosol particles of the overspray aerosol is continued following the end of the spray application via the spray nozzle for a period in the range of 0.5 to 45 minutes, preferably for a period in the range of 1.0 to 30.0 minutes.

17. The method according to any one of the preceding claims, **characterized in that**
the horizontal distance of the opening of the suction device from the spray nozzle is at least 50 cm, in particular at least 75 cm, and/or the, in particular shortest, distance of the opening of the suction device from the shortest connection line between the spray nozzle and the substrate surface to be coated is at least 50 cm, in particular at least 75 cm.

18. Use of the method according to any one of the preceding claims for the development and/or optimization of spray techniques and/or spray devices for the application of coating compositions, in particular of water-based or solvent-based coating compositions, and/or for the development and/or optimization of coating compositions provided for the spray application, in particular of water-based or solvent-based varnishes, and/or for the, in particular instantaneous, quantification of overspray aerosols with regard to the number and size of the aerosol particles present therein and/or for the comparison of the overspray aerosols of at least two, in particular of a multiplicity of, coating compositions which are suitable for the spray application, in particular of water-based or solvent-based varnishes.

19. Use according to Claim 18, **characterized in that**
the development and/or optimization of spray techniques and/or spray devices and/or of coating compositions comprises the reduction of the amount of overspray aerosols.

## Revendications

1. Procédé de détermination, en particulier pratiquement instantanée, de la taille de particule et/ou de la quantité de particules d'aérosol, en particulier de la distribution des tailles de particules d'aérosol, dans des aérosols de surpulvérisation lors de l'application par pulvérisation de matériaux de revêtement, comprenant les étapes suivantes :
- mise à disposition d'une masse de revêtement, d'un dispositif de pulvérisation comprenant une buse de pulvérisation, d'un dispositif d'aspiration, en particulier d'une trémie d'aspiration, avec une ouverture comprenant une surface d'aspiration, une pompe d'aspiration, un système de mesure, comprenant un capteur optique, un tuyau à échantillons pour l'amenée de particules d'aérosol du dispositif d'aspiration au système de mesure, et une unité d'analyse,
- raccordement du dispositif d'aspiration au système de mesure au moyen du tuyau à échantillons,
- raccordement du système de mesure à l'unité d'analyse,
- positionnement de la buse de pulvérisation du dispositif de pulvérisation dans une chambre de mesure, notamment fermée, à un premier intervalle d'une surface de substrat à recouvrir de la masse de revêtement, ayant un bord inférieur et un bord supérieur,
- positionnement du dispositif d'aspiration à un deuxième intervalle de la surface de substrat à revêtir,
- actionnement du dispositif de pulvérisation, de sorte que la masse de revêtement soit appliquée par la buse de pulvérisation sur la surface de substrat à revêtir sous la forme d'un jet d'aérosol comprenant un jet primaire d'aérosol,
- aspiration de l'aérosol de surpulvérisation par le dispositif d'aspiration au moyen de la pompe d'aspiration et transport de particules d'aérosol de l'aérosol de surpulvérisationvers le système de mesure au moyen du tuyau à échantillons,
- détection des particules d'aérosol dans le système de mesure pendant une durée de mesure et transmission des signaux détectés à l'unité d'analyse,
- analyse des signaux détectés dans l'unité d'analyse.

2. Procédé selon la revendication 1, **caractérisé en ce que**
le système de mesure comprend une source lumineuse, une cellule d'écoulement et un photo détecteur.

3. Procédé selon la revendication 2, **caractérisé en ce que**
la source lumineuse comprend ou constitue une diode laser à semi-conducteur, préférentiellement à optique intégrée pour la génération de faisceaux lumineux parallèles.

4. Procédé selon l'une quelconque des revendications précédentes, **caractérisé en ce que** le dispositif d'aspiration est disposé de telle manière que son ouverture est située en dehors du jet primaire de la buse de pulvérisation.

5. Procédé selon l'une quelconque des revendications précédentes, **caractérisé en ce que** sont déterminées des particules d'aérosol ayant une taille de particule comprise entre 0,1 µ et 40 µm, préférentiellement comprise entre 0,3 et 20 µm.

6. Procédé selon l'une quelconque des revendications précédentes, **caractérisé en ce que** la détermination de la taille des particules d'aérosol est fondée sur le principe de mesure de lumière diffusée ou le principe d'extinction.

7. Procédé selon l'une quelconque des revendications précédentes, **caractérisé en ce que** la buse de pulvérisation du dispositif de pulvérisation est orientée de telle manière que le jet primaire du jet d'aérosol est dirigé sensiblement perpendiculairement à la surface de substrat.

8. Procédé selon l'une quelconque des revendications précédentes, **caractérisé en ce que** la buse de pulvérisation est distante d'un intervalle compris entre 0,1 et 0,5 m, en particulier entre 0,15 et 0,3 m, de la surface de substrat à revêtir et/ou **en ce que** la surface d'aspiration du dispositif d'aspiration est distante d'un intervalle horizontal compris entre 0,5 et 3,0 m, en particulier entre 1,0 et 1,9 m, de la surface de substrat à revêtir et/ou **en ce que**
la surface d'aspiration de la trémie d'aspiration est située à une hauteur comprise entre 0 et 2,0 m, préférentiellement située à une hauteur comprise entre 0,5 et 1,0 m, respectivement calculée depuis le bord de la surface de substrat à revêtir, et/ou **en ce que**
la chambre de mesure s'étend entre 1,0 et 3,0 m, en particulier entre 1,5 et 2,5 m, des deux côtés du jet primaire d'aérosols de la buse de pulvérisation et/ou **en ce que** par rapport à la buse de pulvérisation, la chambre de mesure s'étend entre 1,0 et 3,0 m, préférentiellement entre 1,5 et 2,5 m, au-delà de la surface d'aspiration de la trémie d'aspiration et/ou **en ce que**
la chambre de mesure a une hauteur comprise entre 2,0 et 3,5 m, préférentiellement entre 2,25 et 3,0 m.

9. Procédé selon l'une quelconque des revendications précédentes, **caractérisé en ce que** la température dans la chambre de mesure est maintenue constante pendant la durée de mesure.

10. Procédé selon l'une quelconque des revendications précédentes, **caractérisé en ce que** l'humidité relative de l'air dans la chambre de mesure est réglée à une valeur initiale définie au début de la durée de mesure.

11. Procédé selon l'une quelconque des revendications précédentes, **caractérisé en ce que** la température dans la chambre de mesure est comprise entre 10 et 22 °C, préférentiellement entre 12 et 20 °C, et/ou **en ce que**
l'humidité relative de l'air dans la chambre de mesure est comprise entre 0 et 70%, préférentiellement entre 40 et 60 %, en particulier au début de la durée de mesure.

12. Procédé selon l'une quelconque des revendications précédentes, **caractérisé en ce que** la détection de particules comprend la détermination du nombre et/ou de la taille des particules.

13. Procédé selon l'une quelconque des revendications précédentes, **caractérisé en ce que** la détection de particules d'aérosol, en particulier de la quantité et/ou de la taille des particules, est commencée, préférentiellement pendant une durée comprise entre 0,5 et 3 minutes, tout particulièrement entre 1,0 et 2,0 minutes, avant que commence l'application par pulvérisation au moyen de la buse de pulvérisation sur la surface de substrat à revêtir.

14. Procédé selon l'une quelconque des revendications précédentes, **caractérisé en ce que** la quantité de particules d'aérosol ayant une taille de particule comprise entre 0,3 et 20 µm est déterminée, ou **en ce que** la plage de taille de particule de 0,3 à 20 µm est subdivisée en au moins deux, en particulier en une pluralité d'intervalles de taille de particule, et la quantité de particules est déterminée par intervalle de taille de particule et/ou **en ce que** la quantité de particules ayant une taille de particule supérieure à 20 µm est déterminée.

15. Procédé selon l'une quelconque des revendications précédentes, **caractérisé en ce que** que le débit d'air aspiré pendant la mesure sur la surface d'aspiration de la trémie d'aspiration est compris entre 0,1 et 35,0 l/min, préférentiellement entre 25,0 et 30,0 l/min.

16. Procédé selon l'une quelconque des revendications précédentes, **caractérisé en ce que** la détection de particules de l'aérosol de surpulvérisation est poursuivie à l'issue de l'application par pulvérisation au moyen de la buse de pulvérisation pendant une durée comprise entre 0,5 et 45 minutes, préférentiellement pendant une durée comprise entre 1,0 et 30,0 minutes.

17. Procédé selon l'une quelconque des revendications précédentes, **caractérisé en ce que** l'intervalle horizontal entre l'ouverture du dispositif d'aspiration et la buse de pulvérisation est d'au moins 50 cm, en particulier d'au moins 75 cm, et/ou **en ce que** l'intervalle, en particulier minimal, entre l'ouverture du dispositif d'aspiration et la ligne de jonction la plus directe entre la buse de pulvérisation et la surface de substrat à revêtir est d'au moins 50 cm, en particulier d'au moins 75 cm.

18. Utilisation du procédé selon l'une quelconque des revendications précédentes pour le développement et/ou l'optimisation de techniques de pulvérisation et/ou de dispositifs de pulvérisation pour l'application de masses de revêtement, en particulier de masses de revêtement à base d'eau ou à base de solvants, et/ou pour le développement et/ou l'optimisation de masses de revêtement prévues pour l'application par pulvérisation, en particulier de peintures à base d'eau ou à base de solvants, et/ou pour la quantification, en particulier instantanée, d'aérosols de surpulvérisation, relativement au nombre et à la taille des particules d'aérosol qui y sont présentées et/ou pour la comparaison des aérosols de surpulvérisation d'au moins deux, en particulier d'une pluralité de masses de revêtement appropriées pour l'application par pulvérisation, en particulier de peintures à base d'eau ou à base de solvants.

19. Utilisation selon la revendication 18, **caractérisée en ce que**
le développement et/ou l'optimisation de techniques de pulvérisation et/ou de dispositifs de pulvérisation et/ou de masses de revêtement comprennent la réduction de la quantité d'aérosols de surpulvérisation.
